# EUROPEAN PATENT APPLICATION

(11) **EP 3 505 627 A1**
(43) Date of publication of application: **03.07.2019**
(21) Application number: 17382920.1
(22) Date of filing: 28.12.2017
(51) Int. Cl.: C12N 9/96, B82Y 30/00, C01G 49/00

(54) **BIONANOHYBRID MATERIAL, PROCESS FOR PREPARING SAME AND USE THEREOF**

(71) Applicant: Consejo Superior De Investigaciones Científicas (CSIC), 28006 Madrid (ES); Samsung Electronics Co., Ltd., Gyeonggi-do 16677 (KR)
(72) Inventor: PALOMO CARMONA, José Miguel, 28006 MADRID (ES); BENAVENTE RUBIO, Rocío, 28006 MADRID (ES)
(74) Representative: Pons Ariño, Angel

(57) **Abstract**

The present invention relates to synthesis of high stable heterogeneous protein-iron (II) carbonate nanowires (FeCO₃-NWs) bionanohybrids. They were prepared by the use of an enzyme in aqueous bicarbonate solution and iron (II) salt at room temperature and under air. The enzyme induced the *in situ* formation of the FeCO₃ NWs on the protein network. The addition of NaBH₄ as reducing agent permitted to achieve FeCO₃ NWs with a diameter about 5 nm and length about 40 nm. These new bionanohybrids showed an excellent stability to the oxidation almost conserving the catalytic capacity in the degradation of organic pollutants after 30 days.

## Description

The invention relates to a high stable heterogeneous bionanohybrid material comprising protein and iron (II) carbonate nanowires (FeCO₃-NWs), process for the synthesis thereof and its use as a catalyst.

### STATE OF ART

Iron is the most abundant metal in the planet, so being relative inexpensive, relatively nontoxic (considered by the regulatory authorities a "metal with minimum safety concern)(Medicines Agency, Guideline on the Specification Limits for Residues of Metal Catalysts or Metal Reagents, EMEA/ CHMP/SWP/4446/2000, London, February 21, 2008) in comparison with other precious metals. Thus, iron (Fe) is one of the mostly used metals for industrial applications.
Iron catalysis has gained an extraordinary attention in organic synthesis in the last years (Bauer, I., Knölker, H.J. Iron Catalysis in Organic Synthesis. Chem. Rev., 2015, 115, 3170) and the design and development of new kind of iron catalysts is highly desirable.

In particular, iron nanostructures have been strongly developed in the last years with different interesting properties (Nor, Y.A., Zhou, L., Meka, A.K., Xu, C., Niu, Y., Zhang, H., Mitter, N., Mahony, D., Yu, C. Engineering Iron Oxide Hollow Nanospheres to Enhance Antimicrobial Property: Understanding the Cytotoxic Origin in Organic Rich Environment. Adv. Func. Mat. 2016, 26, 5408; Gao, R., Zhang, H., Yan, D. Iron diselenide nanoplatelets: Stable and efficient water-electrolysis catalysts, Nano Energy, 2017,31, 90).

Magnetite (Fe₃O₄) nanoparticles are by far the most studied phase, and are applied in different areas, mainly with biomedical purpose (Arami, H., Khandhar, A., Liggitt, D., Krishnan, K. M. In vivo delivery, pharmacokinetics, biodistribution and toxicity of iron oxide nanoparticles. Chem. Soc. Rev. 2015,44, 8576).

However, the high surface-to-volume ratio of nanomaterials compared to bulk materials generally makes them attractive candidates for their application as catalysts (Shylesh, S., Schnemann, V., Thiel, W. R. Angew. Chem. Int. Ed. 2010, 49, 342).

In this way, the particular conditions of synthesizing Fe nanostructures are critical in term of the particle size but also particle morphology and surface area which exerts tremendous impact on their catalytic properties (Polshettiwar, V., Asefa, T. in Nanocatalysis: Synthesis and Applications, eds. Polshettiwar, V., Asefa, T., Ch 1-9, Wiley, 2013).

Many different strategies have been described in the preparation of iron nanostructures, where depending on the metal source and experimental conditions the corresponding iron species and nanostructure is obtained. In this way the most typical iron species which can be obtained as nanoparticles are iron oxides hematite (α-Fe₂O₃), maghemite (γ-Fe₂O₃), magnetite (Fe₃O₄), iron hydroxide (FeOOH) and in particular cases, α-Fe. This last iron specie is quite difficult to obtain because of the extremely sensitivity of iron to oxidation under air conditions, exhibiting a core-oxide shell structure inevitably.

Many of these process involved the application of hard conditions (e.g. high temperatures or the presence of organic solvent) or the necessity of using special equipment for the production (WO 2014132106 A1; Reddy, L.H., Arias, J.L., Nicolas, J., Couvreur, P. Magnetic nanoparticles: Design and characterization, toxicity and biocompatibility, pharmaceutical and biomedical applications. Chem. Rev. 2012, 112, 5818). Therefore, the application of new green technologies where the iron nanostructures can be synthesized at mild conditions and simple process where no additional equipment is required for the synthesis of the nanoparticles would represent a tremendous impact.

Also the particle size and morphology have an important influence in the catalytic potential. Fe and Fe oxides has been described to be synthesized mainly as nanoparticles with an average size from 10 to 100 mn. Some process about the use of biomolecules (e.g. bacteria or plants) to synthesize of these nanoparticles has been described although in most of cases high size nanoparticles are obtained (Rufus, A., Sreeju, N., Philip, D. Synthesis of biogenic hematite (α-Fe2O3) nanoparticles for antibacterial and nanofluid applications. RSC Advances, 2016, 6, 94206).

Furthermore, of nanoparticles, other iron nanostructures have been recently observed such as 1D-nanomaterials. Acicular 1D iron oxide nanostructures such as needles, nanorods, and nanowires are attracted much attention owing to their enhanced optical, magnetic, catalytic and mechanical properties over spherically shaped nanoparticles.

In particular, magnetic nanowires (NWs) offer potential advantages over magnetic nanoparticles because of their larger surface area to volume ratio and higher magnetic moments originated from their strong shape anisotropy. Indeed, Fe NWs has been successfully applied for example in comparison with other metal NWs with lower impact on cell viability (Martinez-Banderas, A.I., Aires, A., Teran, F.J., Perez, J.E., Cadenas, J.F., Alsharif, N., Ravasi, T., Cortajarena, A.L., Kosel, J. Functionalized magnetic nanowires for chemical and magneto-mechanical induction of cancer cell death, Scientific Reports, 2016, vol. 6).

Several synthetic protocols have been reported in order to perform. Fe NWs, Fe₂O₃ or combination Fe/Fe₂O₃ or Fe/Fe₃O₄ nanowires were synthesized with an average diameter of 30 to 100 nm and length of 800 nm to 1-2 µm as the best results in literature(Lupan, O., Postica, V., Wolff, N., Polonskyi, O., Duppel, V., Kaidas, V., Lazari, E., Ababii, N., Faupel, F., Kienle, L., Adelung, R. Localized Synthesis of Iron Oxide Nanowires and Fabrication of High Performance Nanosensors Based on a Single Fe203 Nanowire, Small, 2017, 13; Rui, H., Wei, L., Weiwei, P., Minggang, Z., Dong Z., Fa-shen L. 1 D Magnetic Materials of Fe3O4 and Fe with High Performance of Microwave Absorption Fabricated by Electrospinning Process. Sci. Rep. 2014, 4, 7493; US 8,865,116 B2).

These Fe Nanowires has been used in catalytic process, for example in the degradation of organic molecules (Huang, Q., Cao, M., Ai, Z., Zhang, L. Reactive oxygen species dependent degradation pathway of 4-chlorophenol with Fe@Fe2O3 core-shell nanowires. Applied Catalysis B: Environ, 2015, 162, 319) or oxygen evolution reaction (OER) in Li-O₂ batteries (Wang, F., Wu, X., Shen, C., Wen, Z. Facile synthesis of Fe@Fe₂O₃ core-shell nanowires as O₂ electrode for high-energy Li-O₂ batteries, *J. Solid State Electrochem.,* **2016,** *20,* 1831).

However, the synthesis of FeCO₃ nanowires has not been reported so far. The present invention provides a new process to produce of protein-FeCO₃ nanowires bionanohybrid materials showing magnetic properties. The bionanohybrids are efficient catalysts in the degradation of organic compounds.

### DESCRIPTION OF THE INVENTION

The present invention discloses a protein-iron carbonate nanowires bionanohybrid material and the process for preparing thereof in aqueous media and in soft conditions. The iron carbonate nanowires (FeCO₃ NWs) in the bionanohybrid material have an average diameter between 5 and 7 nm and average length of between 40 and 93 nm, showing magnetic properties and high catalytic capacity in the degradation of organic pollutants.

A first aspect of the present invention relates to a bionanohybrid material comprising:
- a matrix comprising an enzyme or protein and
- FeCO₃ nanowires,
wherein the FeCO₃ nanowires are embedded in the matrix.

The FeCO₃ nanowires are homogeneously distributed within the matrix.

In a preferred embodiment of the invention, the matrix is formed by the enzyme *Candida antarctica* lipase.

In a preferred embodiment, the FeCO₃ nanowires have an average length between 40 nm and 93 nm, and/or have an average diameter between 5 nm and 7 nm.

In a more preferred embodiment, the FeCO₃ nanowires have an average diameter of 5 nm.

In an even more preferred embodiment, the FeCO₃ nanowires have an average diameter of 5 nm and an average length of 40 nm.

In another preferred embodiment, the FeCO₃ nanowires have an average length of 59 nm and/or an average diameter of 7 nm.

In a preferred embodiment, the bionanohybrid material having an average diameter of 5 nm is paramagnetic.

The interactions between the matrix and the FeCO₃ nanowires are noncovalent.

In a preferred embodiment, the bionanohybrid material has 47% by weight in Fe, obtained by elemental analysis ICP-OES (inductively coupled plasma optical emission spectrometry).

A second aspect of the present invention relates to a process for preparing protein-FeCO₃ nanowires bionanohybrid material. The process comprises the next steps:
a) addition under stirring of a protein or enzyme and of an iron (II) salt to an aqueous solution of sodium bicarbonate, wherein the pH of the aqueous solution of sodium bicarbonate is between the isoelectric point of the enzyme and 10, not including the isoelectric point of the enzyme, and wherein the isoelectric point of the protein or enzyme is below 10,
b) incubation of the mixture obtained in step a) for a time between 14-16 h to obtain a bionanohybrid material (non-reduced bionanohybrid material).

Incubation means that the mixture is left under stirring for the specified time.

In a preferred embodiment, the bionanohybrid material obtained in step b) (also called in the present invention "non-reduced bionanohybrid material"), which is obtained in solid form in the suspension/mixture, is collected, washed with water and dried. This bionanohybrid material comprises a matrix comprising an enzyme or protein and FeCO₃ nanowires embedded in the matrix, wherein the FeCO₃ nanowires have an average length of 59 nm and an average diameter of 7 nm.

The protein or enzyme induces the formation of nanowires. The protein or enzyme allows the homogeneous distribution of the nanowires and avoids the formation of aggregates.

In a preferred embodiment, the process includes a step c) comprising the addition of sodium borohydride (reduction step) to the mixture obtained in step b) to form a bionanohybrid material (also called in the present invention "reduced bionanohybrid material") in solid form, including smaller nanowires than the bionanohybrid material obtained in b), and collection of the solid formed in step c), washing with water and drying thereof.

The combination of using a protein or enzyme for inducing the nanostructure formation and the use of sodium bicarbonate allows to synthesize for the first time iron (II) carbonate (siderite) nanowires. The reducing step (addition of sodium borohydride) permits to achieve FeCO₃ NWs smaller than those obtained in step b) and showing magnetic properties. The bionanohybrid material obtained in step c) comprises a matrix comprising an enzyme or protein and FeCO₃ nanowires embedded in the matrix, wherein the FeCO₃ nanowires have an average length between 40 nm and 93 nm and an average of 5 nm. More preferably, the bionanohybrid material obtained in step c) comprises FeCO₃ nanowires having an average diameter of 5 nm and an average length of 40 nm.

The bionanohybrid material obtained in step c) presents paramagnetic properties.

The pH of the aqueous solution of sodium bicarbonate in step a) must be above the isoelectric point of the enzyme and up to 10. Above pH 10, the synthesis of iron oxide nanoparticles is favored. The isoelectric point of the enzyme cannot be above 10.

In a preferred embodiment, the bionanohybrid material obtained in step b) or c) is collected, washed with water and dried.

The solution in step a) is stirring for avoiding iron oxidation. Preferably, the stirring is carried out at between 340-380 rpm, more preferably at 380 rpm.
If the stirring speed is inferior to the above-mentioned range, the bionanohybrid material which is formed in solid form, would be deposited on the bottom so the bionanohybrid material would not be well-synthetized. On the contrary, if the stirring speed is higher than the above-mentioned range, oxidated side product would be formed.

In a preferred embodiment, in step a) is added the enzyme *Candida antarctica* lipase.

In a preferred embodiment, the amount of enzyme or protein added in step a) is between 2.5 mg and 5 mg of enzyme or protein per 10 mL of the aqueous solution.

In a preferred embodiment, in step a) is carried out at room temperature (20-25°C) and under air.

In a preferred embodiment, in step a) the addition of enzyme or protein is carried out at pH 10.

In a preferred embodiment, in step a), iron salt concentration ranges from 5 to 100 mg/ml in the aqueous solution; more preferably, 10 mg/ml.

Preferably, the iron (II) salt is the sulfate of iron (II) and ammonium: (NH₄)₂Fe(SO₄)₂.

In a preferred embodiment, the time of the incubation (step b)) is 16 h.

In a preferred embodiment, in step c) sodium borohydride is added to a final concentration of at least 0.12 M in the mixture, more preferably, between 0.12-0.15 M.

In step c), the mixture of reaction is preferably allowed to react between 15 and 360 min, more preferably 30 min and even more preferably 15 min.

In a preferred embodiment, the bionanohybrid material obtained in step b) or c) that has been collected, washed with water and dried is lyophilized. Preferably, it is lyophilized for a time between 16 and 18 h., more preferably 16 h.

The present invention also relates to a bionanohybrid material obtained by the process (in step b) or in step c)) described in the second aspect of the invention. The bionanohybrid material obtained by the process of the invention has the features of the bionanohybrid material described in the first aspect of the present invention.

Another aspect of the present invention refers to the use of the bionanohybrid materials described in the present invention as catalysts.

In a preferred embodiment, the bionanohybrid materials described in the present invention are used as catalysts for degrading organic pollutants, preferably phenolic compounds.

More preferably, the bionanohybrid materials (both the reduced and the non-reduced bionanohybrid material) are used for the degradation of 4-aminophenol (pAP). This degradation firstly gives rise to different intermediate compounds, and finally to CO₂ and H₂0.

More preferably, the reduced bionanohybrid material of the present invention is used for the reduction of 4-nitrophenol (pNP) to 4-aminophenol (pAP).

pNP contamination of surface and ground-water has gradually increased due to the excessive consumption of dyes, pesticides, and pharmaceuticals from industrial and agricultural activities. United States Environmental Protection Agency set a guideline restricting the contaminant level of p-NP bellowing 10 ng/L in natural water.

pAP is the hydrolytic product of acetaminophen (paracetamol) and it has been reported to have significant nephrotoxicity and teratogenic effects.

Testing of the present invention showed that contaminants (150 mg/L) p-nitrophenol and p-aminophenol (100 mg/L) were rapidly degraded with only a very low amount of the reduced bionanohybrid material (1 g/L).

The term "nanowire" in the present invention refers to an elongated nanostructure formed in a wire shape wherein the average length is between 40 to 93 nm and the average diameter is between 5 to 7 nm. Typically, an aspect ratio (diameter: length) of the nanowires of the present invention ranges from.5:40 to 7:93.

The term "bionanohybrid material" in the present invention refers to a material comprising a protein or enzyme and wires with nanometric scale ("nanowires" as defined above).

The term "embedded" in the present invention when referring to the FeCO₃ nanowires means that FeCO₃ nanowires are disposed within the matrix, so they are surrounded by the matrix material.

In summary, this invention provides a simple and green technology to produce for the first time highly active, stable and reusable novel nanocatalysts; bionanohybrids constitute of very small iron carbonate nanowires *in situ* synthesized in a protein or enzyme matrix. These nanohybrids (nanohybrid material) present also magnetic properties, which permit a rapid recovering of the catalyst.

Unless otherwise defined, all technical and scientific terms used herein have the same meaning as commonly understood by one of ordinary skilled in the art to which this invention belongs. Process and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### BRIEF DESCRIPTION OF THE DRAWINGS

**FIG. 1****:** X-ray diffraction (XDR) spectrum of the bionanohybrid material obtained in example 2 of the present invention.
**FIG. 2****:** Transmission electron microscopy (TEM) images of FeCO₃ nanowires of bionanohybrid material obtained in example 2 of the present invention.
**FIG. 3****:** Chemical scheme of the transformation of p-nitrophenol (pNP) into p-aminophenol (pAP).
**FIG. 4****:** X-ray diffraction (XDR) spectrum of the bionanohybrid material **CAL-B-FeCO3NWs-15** obtained in example 2 of the present invention.
**FIG. 5****:** TEM picture of the bionanohybrid material **CAL-B-FeCO3NWs-15** obtained in example 1 of the present invention.
**FIG. 6****:** X-ray photoelectron spectroscopy (XPS) spectra of (a) Fe2p and (b) O1s for iron carbonate in **CAL-B-FeCO3NWs-15** bionanohybrid obtained in example 1 of the present invention. These spectra and XDR spectra of fig. 4 show that the Fe species in the nanowires is FeCO₃.
**FIG. 7****:** Reduction of pNP catalyzed by of the bionanohybrid **CAL-B-FeCO₃NWs-15** in aqueous media after 20 sg of reaction.
**FIG. 8****:** Reduction of pNP catalyzed by others bionanohybrid **(CAL-B-FeCO₃NWs-30, CAL-B-FeCO₃NWs-45, CAL-B-FeCO₃NWs-60, CAL-B-FeCO₃NWs-360)** in aqueous media.
**FIG. 9****:** Comparison of the XDR spectrum of **CAL-B-FeCO₃NWs** bionanohybrid obtained in example 2 of the present invention from the preparation (day 1) to 30 days after.
**FIG. 10****:** Comparison of the XDR spectrum of the bionanohybrid **CAL-B-FeCO3NWs-15** from the preparation (day 1) to 30 days after.
**FIG. 11****:** TEM analysis of the bionanohybrids after 30 days' store. A) bionanohybrid material not treated with NaBH₄ **(CAL-B-FeCO₃NWs).** B) bionanohybrid material treated with NaBH₄ for 15 min **(CAL-B-FeCO₃NWs-15).**

### EXAMPLES

### General

*Candida antartica* B lipase (CAL-B) solution was from Novozymes (Denmark) (the concentration of the enzyme in the commercial solution is 5 mg /ml). Ammonium iron (II) sulfate hexahydrate [(NH₄)₂Fe(SO₄)₂ x 6H₂O (Mohr's salt)], hydrogen peroxide (33%), sodium bicarbonate and sodium borohydride were purchased by Sigma-Aldrich. Acetonitrile HPLC grade was purchased by Scharlab.

Inductively coupled plasma atomic emission spectrometry (ICP-AES) was performed on a Perkin Elmer OPTIMA 2100 DV equipment. The X-Ray diffraction (XRD) pattern was obtained using a Texture Analysis Diffractometer D8 Advance (Bruker) with Cu Kα radiation. X-ray photoelectron analysis (XPS) was carried out on SPECS GmbH spectrometer equipped with Phoibos 150 9MCD energy analyzer. A no monochromatic magnesium X-ray source with a power of 200 W and voltage of 12 kV was used. The transmission electron microscopy (TEM), high resolution TEM microscopy (HRTEM) analysis were performed on a JEOL 2100F microscope equipped with an EDX detector INCA x-sight (Oxford Instruments). The scanning electron microscopy (SEM) imaging was performed on a TM-1000 (Hitachi) microscope. To recover the biohybrids, a Biocen 22 R (Orto-Alresa, Spain) refrigerated centrifuge was used. The spectrophotometric analyses were run on a V-730 spectrophotometer (JASCO, Japan). HPLC spectrum P100 (Thermo Separation products) was used. Analyses were run at 25°C using an L-7300 column oven and a UV6000LP detector.

### Example 1: General synthesis bionanohybrid materials according to the present invention including the reduction step

3.6 mL of commercial *Candida antarctica* lipase solution was added to 60 mL sodium bicarbonate buffer 0.1M pH =10 in a 100 mL glass bottle containing a small magnetic bar stirrer (12x4.5 mm). The solution is stirring in a magnetic agitator OVAN Micromix at 380 r.p.m. for 2 min. Then 600 mg of Fe (NH₄)₂(SO₄)₂* 6H₂O (10 mg/ml) was added to the protein solution and it was maintained for 16 hours. After the first 30 min incubation, the solution turned cloudy (greenish gray) and the pH solution was measured indicating a decrease from 10 to 7.

After 16 h incubation, the solution turned very dark green. The color is indicative, if turn orange means that oxidation was produced.

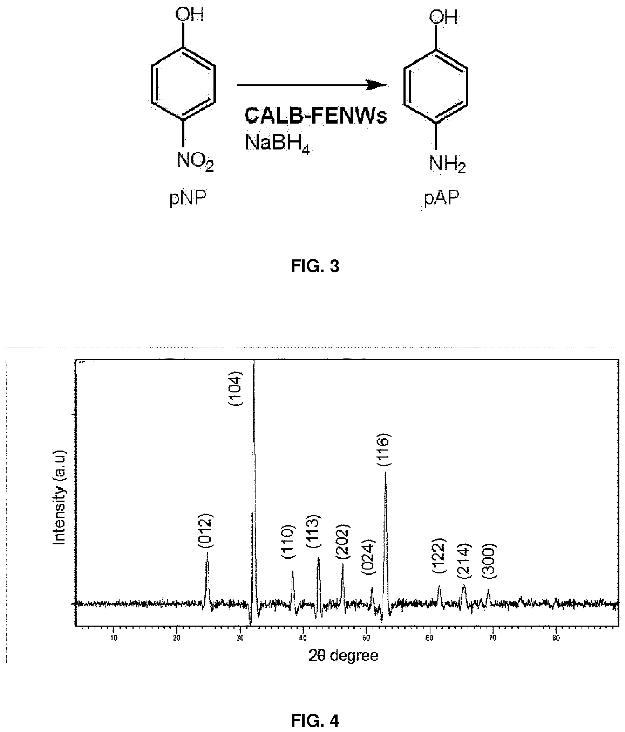

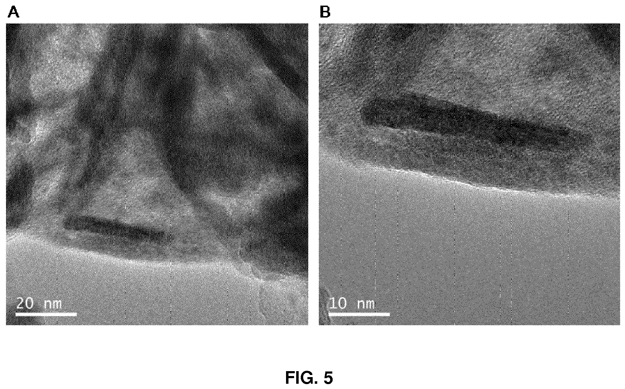

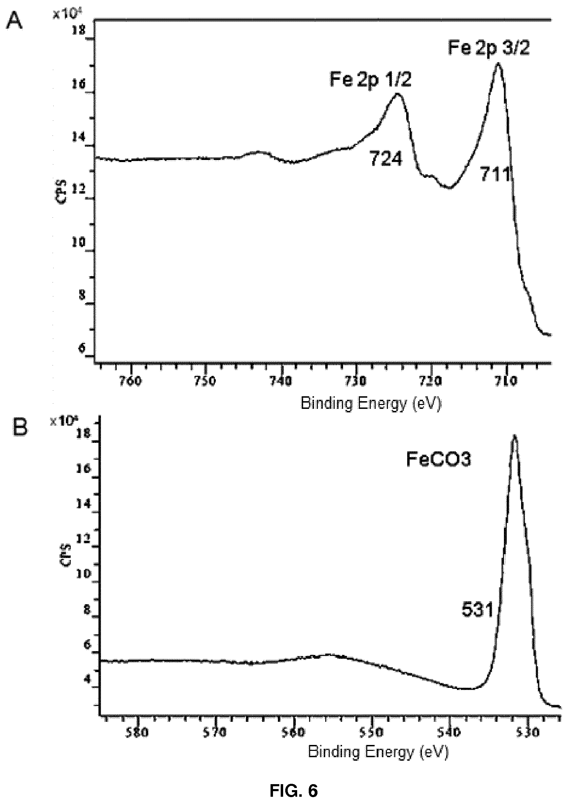

Then, after these 16 h, 6 mL of NaBH₄ (300 mg) aqueous solution (1.2 M) was added to the cloudy solution (in two times of 3 mL) obtaining a final concentration of 0.12 M of sodium borohydride in the mixture. The solution turned rapidly black and, the mixture was reduced during different times depending on the bionanohybrid prepared. The reducing step with sodium borohydride was carried out for 15, 30, 60, 90 and 360 minutes to obtain the bionanohybrid materials: CAL-B-FeCO₃NWs-15, CAL-B-FeCO₃NWs-30, CAL-B-FeCO₃NWs-60, CAL-B-FeCO₃NWs-90, CAL-B-FeCO₃NWs-360, respectively. The obtained mixture was centrifuged at 8000 rpm for 5 min, (11 mL per falcon type tube). The generated pellet was re-suspended in 15 mL of water. The pH of the supernatant solution was measured to be approximately 9. It was centrifuged again at 8000 r.p.m for 5 min and the supernatant removed. The pH of the supernatant solution was measured again, given a pH value of 7. The process was repeated once more. The pH of the final supernatant solution should be 5 to avoid residues of sodium borohydride and its interaction with the freeze-drying. Finally, the supernatant was removed and the pellet of each falcon was re-suspended in 2 mL of water, collected all solutions in a round-bottom flask, frozen with liquid nitrogen and lyophilized for 16 hours. Characterization of the different bionanohybrids was performed by XDR, XPS, TEM analysis. Also these were repeated after 30 days of preparation.
When the reduction was performed at 15 min, small nanowires (approx. 5 nm diameter x 40 nm long) of FeCO₃ were formed and determined by TEM, XDR (Figure 4-5) and XPS (Figure 6). This bionanohybrid material (CAL-B-FeCO₃NWs-15) presented excellent magnetism against a magnetic field.

The reduction was also performed at 30, 45, 60 and 360 minutes. The XDR demonstrated that the iron species in the different bionanohybrids did not change, being a stable siderite (FeCO₃). However, TEM analysis (and using software INCA x-sight) revealed the generation of longer size nanowires, from around 40 to 102 nm, increasing with the reducing time although conserving the diameter (Table 1).

**Table 1. The dependence of FeCO₃ nanowire size on different reducing times in the presence of sodium borohydride.**

| | | | | | | |
|---|---|---|---|---|---|---|
| Reducing time (min) | 0 | 15 | 30 | 45 | 60 | 360 ^{a} |
| Diameter of nanowires/nm | 7±3 | 5±1 | 5±1 | 5±1 | 5±1 | 5±1 |
| Length of nanowires/nm | 59±9 | **40±5** | 55±5 | 90±7 | 91±7 | 93±8 |

| | | | | | | |
|---|---|---|---|---|---|---|
| ^{a} iron nanoparticles were also observed. | | | | | | |

### Example 2: Synthesis bionanohybrid material of the present invention without including the reduction step

The same procedure as example 1 was followed, except the addition of sodium borohydride. Instead of that, after 16 h incubation, the obtained mixture was centrifuged at 8000 rpm for 5 min, (11 mL per falcon type tube). The generated pellet was re-suspended in 15 mL of water. Finally, the supernatant was removed and the pellet of each falcon was re-suspended in 2 mL of water, collected all solutions in a round-bottom flask, frozen with liquid nitrogen and lyophilized for 16 hours.

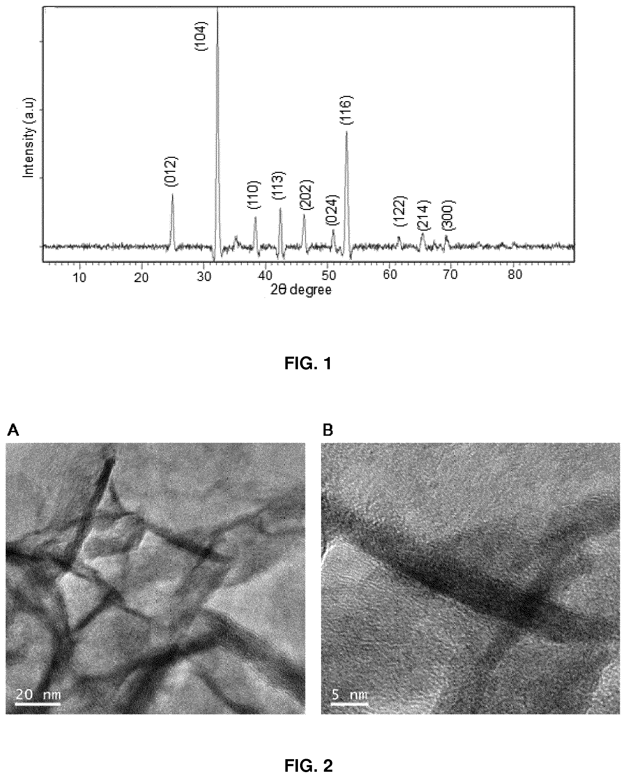

Characterization of the bionanohybrid obtained (CAL-B-FeCO₃NWs) was performed by XDR, TEM analysis (see figures 1 and 2). The spectra show the formation of FeCO₃ nanowires with a size approx. 7 nm diameter x 59 nm long induced by the protein matrix (Figure 2). This hybrid did not give evidence of magnetism in the presence of a magnetic field.

### Example 3: Catalytic Reduction of 4-nitrophenol (pNP) to 4-aminophenol (pAP) by the bionanohybrid materials of the present invention

To an aqueous solution of p-nitrophenol (pNP) (1 mM; 2 mL), solid NaBH₄ (3 mg) was added to reach a final concentration of 0.04 M (The typical catalytic reaction was performed by adding an excess of NaBH₄ (0.04 M) to ensure its constant concentration throughout the reaction and, therefore, to apply a pseudo-first-order kinetic with respect to the pNP to an aqueous solution of the substrate in the presence of catalysts). In these conditions, upon the addiction of NaBH₄, the initial absorbance band of the solution of pNP undergoes to an immediate shift from 317 to 400 nm due to the formation of 4-nitrophenolate ions. Immediately after that, 3 mg of the different bionanohybrids were added under gentle stirring at 25 °C in an orbital shaker. The reaction progress was monitored by taking out an aliquot of the solution (0.1 mL) at different times, diluting it with distilled water (2 mL) and measuring the absorption spectrum between 500 and 300 nm in a quartz cuvette.

The fig. 3 shows the chemical scheme of the transformation of p-nitrophenol (pNP) into p-aminophenol (pAP).

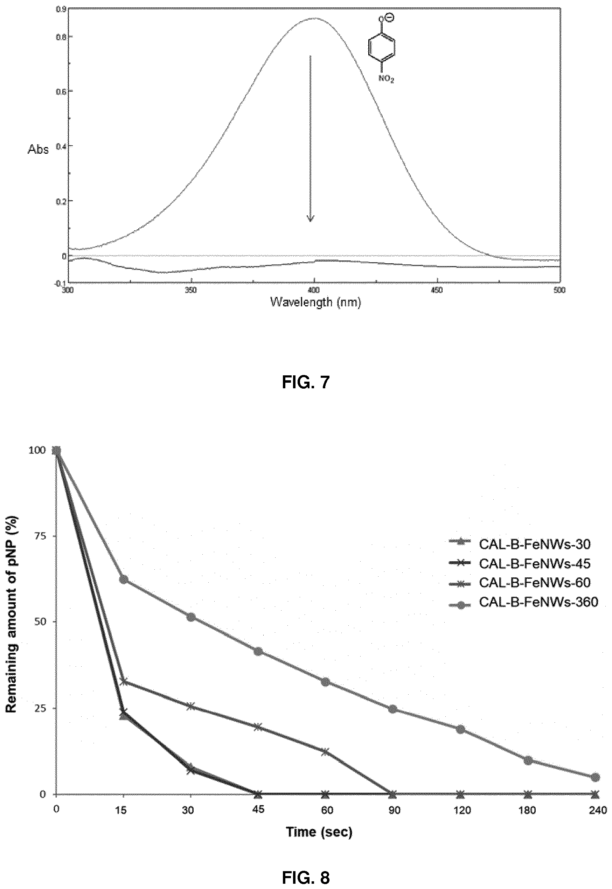

This bionanohybrid material (CAL-B-FeCO₃NWs-15) showed the higher catalytic capacity to complete convert p-NP to p-AP within 20 seconds (3 mg catalyst, 2 ml, 1 mM pNP) (Figure 7).

The activity of the bionanohybrids was lower as the reduction time increased. No differences in catalytic efficiency were observed between the reduced catalysts for 30 or 45 min, but 33% slower rate was obtained compared with the activity achieved with CAL-B-FeCO₃NWs-15, full conversion was achieved after 45 seconds. The CAL-B-FeCO₃NWs-60 achieved full reduction after 90 seconds whereas a remaining 5% of pNP was not still converted after 240 seconds for the CAL-B-FeCO₃NWs-360 (6h reducing incubation) (Figure 8). Therefore, these results demonstrated that a short-time reducing step strongly improved the catalytic efficiency of these CAL-B-FeCO₃NWs bionanohybrids for these pNP degradation where the non-reduced biohybrid was not efficient

The catalytic activity of the bionanohybrid material obtained in example 2 (CAL-B-FeCO₃NWs) in the degradation of p-NP at 150 mg/L was tested. No conversion was observed.

### Example 4: Reuse of CAL-B-FeCO₃NWs-15 bionanohybrid in the reduction of pNP

The CAL-B-FeCO₃NWs-15 bionanohybrid was reused six cycles in the reduction of pNP using the conditions described above. The catalyst was washed with water once and centrifuged before the next reaction. The fresh catalyst was reused at least 6 reaction cycles in the reduction of pNP and more than 95% activity was conserved after that.

### Example 6: Catalytic Degradation of p-aminophenol (pAP) by bionanohybrid materials of the present invention

2 mg of pAP was dissolved in 18.88 mL solution of distilled water and 0.22 mL of hydrogen peroxide was added. To initialize the reaction, 2 mL of this solution was added to a 7 mL glass bottle containing 3 mg of bionanohybrid gentle stirring at 25 °C in an orbital shaker (320 rpm). Samples (50 µl) at different times were taken and the reaction was followed by HPLC. The samples were diluted 40 times in distilled water before injection. The HPLC column was C8 Kromasil 150x4,6 mm AV-2059. The HPLC conditions used were: an isocratic mixture of 30% acetonitrile and 70% bi-distilled water, UV detection at 275 nm and 225 nm using a Diode array detector, and a flow rate of 0.5 mL/min. Under these conditions, the retention times of pAP was 4.65 min, and for H₂O₂ was 3.3 min.
The reaction was quantitatively followed by HPLC. The CAL-B-FeCO₃NWs-15 (obtained in example 1) and CAL-B-FeCO₃NWs (obtained in example 2) showed similar performance at these conditions and more than 98% degradation of pAP was achieved in 2 min (Table 2). No traces of any compounds were detected by HPLC after 50 min.
These results show that at these conditions both no reduced and reduced bionanohybrid were efficient catalysts in the degradation of this organic pollutant.

**Table 2. Degradation of pAP by CAL-B-FeCO₃NWs bionanohybrids in the presence of 0.5 mM of hydrogen peroxide. The dependence of FeCO₃ nanowire size on different reducing times in the presence of sodium borohydride.**

| Bionanohybrid | pAP Degradation yield | Time (min) |
|---|---|---|
| **CAL-B-FeCO₃NWs** | 98% | 2 |
| **CAL-B-FeCO₃NWs-15** | 99% | 2 |

### Example 7: Stability of the bionanohybrid materials of the present invention

One important point for a possible industrial application of this protein-FeNWs bionanohybrids is the stability against oxidation.

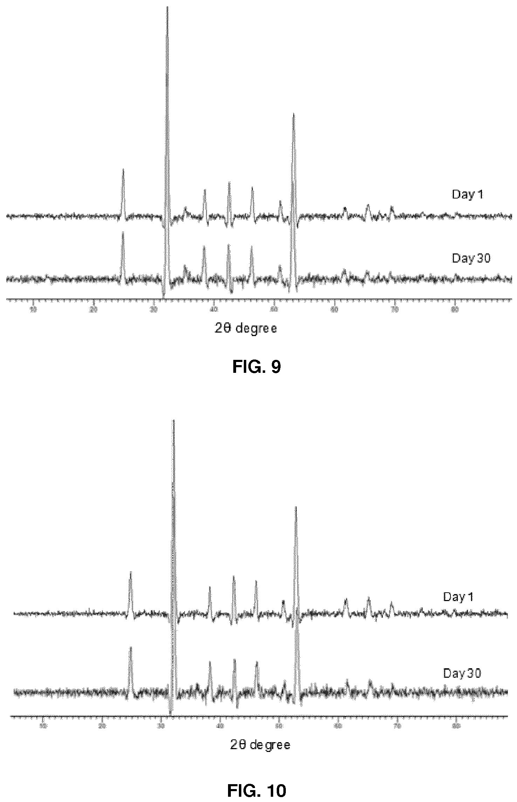

In this term, the CAL-B-FeCO₃NWs and CAL-B-FeCO₃NWs-15 bionanohybrids were stored at air conditions for 30 days. After that time, analysis of XDR showed that no changes in any cases were observed in the iron specie (Figure 9-10).

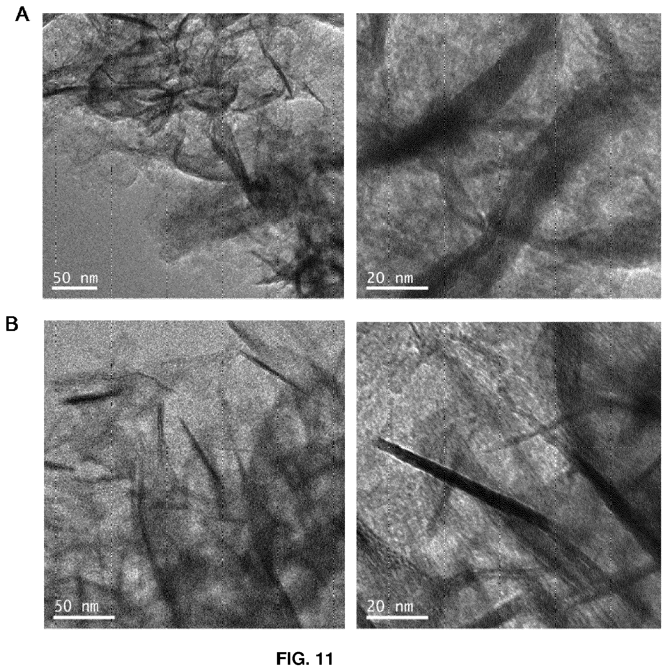

The catalytic efficiency of the CAL-B-FeCO₃NWs-15 in the reduction of pNP still was excellent, with a full conversion of the substrate in 60 seconds. TEM analysis revealed the slight increase of size in the NWs which explained the slight decrease in the efficiency (Table 3, Figure 11).

**Table 3. FeCO₃ nanowire size on the different nanobiohybrids after 30 days.**

| Bionanohybrid | **CAL-B-FeCO₃NWs** | **CAL-B-FeCO₃NWs-15** |
|---|---|---|
| Diameter of nanowires/nm | 8±2 | 6±1 |
| Length of nanowires/nm | 88±14 | 60±6 |

## Claims

1. A bionanohybrid material comprising:
- a matrix formed by an enzyme or a protein and
- FeCO₃ nanowires,
wherein the FeCO₃ nanowires are embedded in the matrix.

2. The material according to claim 1, wherein the matrix is formed by the enzyme *Candida antarctica* lipase.

3. The material according to any of claims 1 or 2, wherein the FeCO₃ nanowires have an average length between 40 nm and 93 nm and/or have an average diameter between 5 nm and 7 nm.

4. The material according to claim 3 wherein the FeCO₃ nanowires have an average length of 59 nm and/or an average diameter of 7 nm.

5. The material according to claim 3 wherein the FeCO₃ nanowires have an average diameter of 5 nm.

6. A process for preparing a bionanohybrid material defined in any of claims 1 to 5, wherein the process comprises at least the following steps:
a) addition under stirring of a protein or enzyme and of an iron (II) salt to an aqueous solution of sodium bicarbonate, wherein the pH of the aqueous solution of sodium bicarbonate is between the isoelectric point of the enzyme and 10, not including the isoelectric point of the enzyme, and wherein the isoelectric point of the protein or enzyme is below 10,
b) incubation of the mixture obtained in step a) for a time between 14-16 h.

7. The process according to claim 6, further comprising a step c) wherein the step c) comprises the addition of sodium borohydride to the mixture obtained in step b).

8. The process according to any of claims 6 or 7, wherein the bionanohybrid material obtained in step b) or c) is collected, washed with water and dried.

9. The process according to any of claims 6 to 8, wherein the enzyme added in the step a) is *Candida antarctica* lipase.

10. The process according to any of claims 6 to 9 wherein the iron (II) salt concentration in step a) ranges from 5 to 100 mg/ml in the aqueous solution.

11. The process according to any of claims 6 to 10 wherein the iron (II) salt is the sulfate of iron (II) and ammonium.

12. The process according to any of claims 6 to 11 wherein the solution in step a) is stirring at speed between 340-380 rpm.

13. The process according to any of claims 6 to 12 wherein the amount of enzyme or protein added in step a) is between 2.5 mg and 5 mg per 10 mL of the aqueous solution.

14. The process according to any of claims 6 to 13 wherein the time of the incubation in step b) is 16 h.

15. The process according to any of claims 7 to 14 wherein, in step c), sodium borohydride is added to a final concentration between 0.12 to 0.15 M in the mixture of reaction.

16. The process according to any of claims 7 to 15 wherein, in step c), the mixture of reaction is allowed to react between 15 and 360 min.

17. The process according to any of claims 8 to 16 wherein the bionanohybrid material that has been collected, washed with water and dried is lyophilized.

18. Use of the material described in any of claims 1 to 5 as a catalyst.

19. Use of the material according to claim 18 for the degradation of 4-aminophenol.

20. Use of the material described in claims 5 as a catalyst for the reduction de 4-nitrophenol to 4-aminophenol.
